# EUROPEAN PATENT SPECIFICATION

(11) **EP 2 826 465 B1**
(45) Date of publication and mention of the grant of the patent: **05.09.2018**
(21) Application number: 14177623.7
(22) Date of filing: 18.07.2014
(51) Int. Cl.: A61K 9/20, A61K 31/445

(54) **Orally disintegrating tablet formulations of donepezil**
Im Mund zerfallende Tablettenformulierungen von Donepezil
Formulations de comprimé à désintégration orale de donépézil

(30) Priority: 19.07.2013 TR 201308801
(43) Date of publication of application: 21.01.2015
(73) Proprietor: Sanovel Ilac Sanayi ve Ticaret A.S., 34460 Istanbul (TR)
(72) Inventor: Türkyilmaz, Ali, 34460 Istanbul (TR); Celik, Devrim, 34460 Istanbul (TR); Aygül, Fatih Cengiz, 34460 Istanbul (TR)
(74) Representative: Sevinç, Erkan

(56) References cited:
- EP-A1- 2 198 857
- EP-A1- 2 468 254
- WO-A1-2004/091585
- WO-A2-2011/115599
- US-A1- 2007 129 402
- US-A1- 2011 060 008
- YAN YI-DONG ET AL: "Preparation and evaluation of taste-masked donepezil hydrochloride orally disintegrating tablets", BIOLOGICAL & PHARMACEUTICAL BULLETIN SEP 2009,, vol. 33, no. 8, 1 January 2010 (2010-01-01), pages 1364-1370, XP009174501, ISSN: 1347-5215
- Anonymous: "NISSO HPC for Pharmaceutical Applications", Internet citation, 1 January 2013 (2013-01-01), XP55354406, Retrieved from the Internet: URL:http://www.dalian-diligence.com/images /HPC Catelog (English) final.pdf [retrieved on 2017-03-14]
- Anonymous: "Public Assessment Report Mutual Recognition Procedure Aricept Evess 5mg Orodispersible Tablets Aricept Evess 10mg Orodispersible Tablets UK/H/182/03-04 UK licence no: PL 10555/0019-20 Eisai Limited", , 13 September 2003 (2003-09-13), XP55354610, Retrieved from the Internet: URL:http://www.mhra.gov.uk/home/groups/par /documents/websiteresources/con2025091.pdf [retrieved on 2017-03-14]

## Description

### Technical Field of the Invention

The present invention relates to orally disintegrating tablet formulations of donepezil hydrochloride comprising magnesium aluminium silicate, and one or more pharmaceutically acceptable excipient and process for preparing such a formulation.

### Background of the Invention

Donepezil hydrochloride is a reversible inhibitor of the enzyme acetylcholinesterase, which is known as (±)-2,3-dihydro-5,6-dimethoxy-2-[[1-(phenylmethyl)-4-piperidinyl]methyl]-1*H*-inden-1-one hydrochloride and its chemical structure is shown in the Formula I.

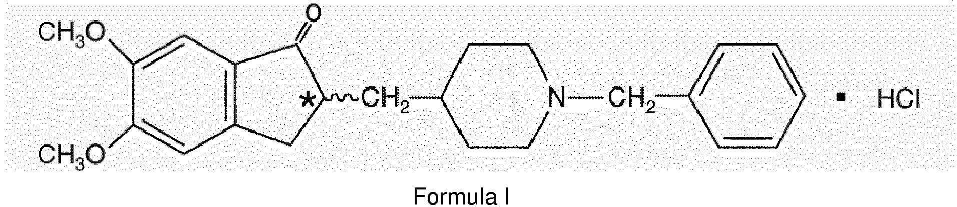

Donepezil hydrochloride is available for oral administration in conventional tablet formulations and orally disintegrating tablet formulations containing 5 or 10 mg of donepezil hydrochloride and indicated for the treatment of dementia and Alzheimer's Disease.

Various formulations and methods are already known for the preparation of orally disintegrating formulations of donepezil or pharmaceutically acceptable salts thereof. EP 2 198 857 A1 discloses oral dispersible tablets comprising donepezil hydrochloride, at least one filler selected from sugars and sugar alcohols, and microcrystalline cellulose. WO 2001/115599 A2 discloses orally disintegrating tablets of donepezil hydrochloride comprising mannitol, crospovidon, sucralose and one or more pharmaceutically acceptable excipient.

However, orally disintegrating formulations are becoming an increasingly important issue in the area of better patient compliance comparative to the conventional solid dosage forms for oral administration such as capsules and tablets, which are the most commonly used. In particular pediatric and geriatric patients, and patients with mental problems such as dementia and Alzheimer's disease, often experience difficulties in swallowing solid dosage forms. Besides, conventional solid dosage forms are not suitable for bedridden or busy and travelling patients, in case the patient may not have easy access to water. Thus, orally disintegrating compositions represent an alternative for such patients and provide for a better patient compliance with recommended pharmaceutical therapies.

Additionally oral administration of the drugs is difficult in patients having concomitant vomiting, nausea or diarrhoea. The orally disintegrating dosage form is one of the advantageous methods to deliver the drugs to such patients. By administering the orally disintegrating dosage forms, faster absorption of the drug occurs through buccal mucosa and it may reduce the first pass metabolism leading to better efficacy of the drug. This dosage form enhances the clinical effects of some drugs by leading to an increase in bioavailability and a reduction in side effects because of avoidance of first-pass liver metabolism.

It is known that developing orally disintegrating compositions is difficult because of several different reasons. A satisfying orally disintegrating dosage form needs to meet number of requirements. Firstly, it has to disintegrate in the oral cavity rapidly. Moreover, a premature release in the mouth could also lead to problems due to the often unpleasant taste of the active ingredient. Finally, these compositions should be very porous and should not be very hard. These porous compositions tend to be very sensitive to humidity so they may have some stability problems.

In order to meet all these above described requirements, careful selection of the excipients plays a significant role and they have to be chosen very carefully.

As a consequence, a need rises for orally disintegrating tablet formulations of donepezil hydrochloride and a process for preparing such formulations that overcome the problems as disintegration, taste and stability. The present invention offers better stability, taste and disintegration rate of orally disintegrating tablet formulations of donepezil hydrochloride. According to the present invention, orally disintegrating tablet formulations of donepezil hydrochloride do not comprise crospovidon in contrast to the prior art, and by using magnesium aluminum silicate, stability problems are solved and surprisingly better stability is gained. Also, specific combination of low viscosity hydroxypropyl cellulose (HPC-SSL) and high viscosity hydroxypropyl cellulose (L-HPC) yields a synergistic effect over the disintegration time and mechanical strength (such as; hardness and friability) of the orally disintegrating tablet formulation.

Advantages and embodiments of the present invention will become apparent from the following description.

### Detailed Description of the Invention

The main object of the present invention is to provide an improved orally disintegrating tablet formulation of donepezil hydrochloride comprising magnesium aluminum silicate which overcomes above described problems with using appropriate excipients.

According to this object the present invention is directed to an orally disintegrating tablet formulation of donepezil hydrochloride that is present in an amount of 1.00 to 10.0 % by weight, comprising magnesium aluminum silicate in an amount of between 1.00 to 15.0 % by weight of the total tablet formulation, and wherein the formulation is crospovidon free, as defined in claim 1. The invention also relates to a process for preparing said orally disintegrating tablet formulation as defined in claim 10.

The amount of donepezil hydrochloride is preferably 1.00 to 5.00 % by weight of the total tablet formulation, and the amount of magnesium aluminum silicate is preferably 2.00 to 10.00 % by weight of the total tablet formulation.

The presence of magnesium aluminum silicate provides better stability for an orally disintegrating tablet formulation of donepezil hydrochloride which does not comprise crospovidon in contrast to prior art. It is found that crospovidon leads to stability problems due to impurities, thus cause to disintegrating problems during its shelf life period. It has surprisingly been found that magnesium aluminum silicate solves the stability problems and leads to desirable stability.

In one embodiment, the orally disintegrating tablet formulation of donepezil hydrochloride comprises hydroxypropyl cellulose with low and high viscosity.

In a further embodiment, the orally disintegrating tablet formulation of donepezil hydrochloride comprises hydroxypropyl cellulose having low viscosity which is between 2.0 and 2.9 mPa.s, (HPC-SSL), and hydroxypropyl cellulose having high viscosity which is between 6.0 and 10.0 mPa.s that is a grade of L-HPC that is LH-11. If the orally disintegrating tablet formulation of donepezil hydrochloride comprises low viscosity hydroxypropyl cellulose, this is HPC-SSL in an amount of between 0.01 to 5.00 % by weight, preferably it is 0.01 to 3.00 % by weight of total tablet formulation. HPC-SSL having the lowest viscosity in all other grades of hydroxypropyl cellulose (HPC) is used as a binder.

Also, the orally disintegrating tablet formulation of donepezil hydrochloride may comprise high viscosity hydroxypropyl cellulose that is L-HPC in an amount of between 1.00 to 20.00 % by weight, preferably it is 1.00 to 10.00 % by weight of total tablet formulation. L-HPC is used as a disintegrant.

In another embodiment, according to the orally disintegrating tablet formulation of donepezil hydrochloride, the ratio of HPC-SSL to L-HPC is between 1:10 and 10:1 by weight, preferably it is between 1:5 and 5:1 by weight, more preferably it is between 1:5 and 1:1 by weight.

It has surprisingly been found that the specific combination of HPC-SSL and L-HPC in the above-mentioned ratios creates a synergistic effect over the the disintegration time, mechanical strength (such as; hardness and friability) and compressibility of the orally disintegrating tablet formulation. L-HPC can absorb water fastly yielding powerful, high degree of swelling and so it leads to rapid disintegration of tablets and also using HPC-SSL with L-HPC provides optimum and desirable disintegration, better mechanical strength and better compressibility since presence of HPC-SSL preventing the immediate and undesirably fast disintegration of orally disintegrating tablet of donepezil hydrochloride. Therefore, due to the synergistic effect of L-HPC and HPC-SSL combination, the composition disintegrates in oral cavity in less than 60 seconds, preferably in less than 30 seconds.

In another embodiment, the orally disintegrating tablet formulation of donepezil hydrochloride comprises mannitol having the average particle size of 160 µm, *Pearlitol 160C®,* and mannitol having the average particle size of 360 µm, *Pearlitol 400DC®.* In this invention, the average particle size is measured by Malvern Particle Size analyzer based on Lazer Diffraction by using dry dispersion method. According to the calculation principle of the analyzer, the volume of the particles is converted into the volume of equivalent sphere and the result is the average diameters of these spheres which is volume moment mean D(4,3) value.

In the orally disintegrating tablet formulation of donepezil hydrochloride, the ratio of mannitol having the average particle size of 160 µm to mannitol having the average particle size of 360 µm is between 1:10 and 10:1 by weight, preferably it is between 1:5 and 5:1 by weight, more preferably it is between 1:5 and 1:1 by weight.

It is found that the specific combination of *Pearlitol 160C®* and *Pearlitol 400DC®* make a contribution to the obtaining better disintegration time and mechanical strength of the orally disintegrating tablet formulation. Therefore the hardness of the tablet is between 5 N to 50 N, preferably it is between 20 N to 30 N.

In another embodiment, the orally disintegrating tablet formulation of donepezil hydrochloride comprises magnesium aluminum silicate in an amount of between 1.00 to 15.00 % by weight, HPC-SSL in an amount of between 0.01 to 5.00 % by weight and L-HPC in an amount of between 1.00 to 20.00 % by weight of total tablet formulation and one or more pharmaceutically acceptable excipient.

The orally disintegrating compositions of this invention may also comprise sucralose as a sweetener to improve patient compliance. In the prior art, it is known that aspartame is used mostly as sweetner but contradictory to the prior art we have found that the effect of sucralose as a sweetner in this formulation, not only helped to improve its taste but also increased the efficacy and the conveniency of the formulation because of its positive effects over the glycemic index. There are lots of disadvantages about aspartame and it has a limited usage if you have to use it every day and also there are several incompatibilities reported in literature and safety problems *(*Handbook of Pharmaceutical Excipients, Reymond C Rowe, Paul J Sheskey, Marian E Quinn, sixth edition, pages 48-50). Thus, sucralose has an important role in this aspect and even if it is used in low amounts it has a synergistic taste improvement with mannitol which is also very important issue in orally disintegrating tablet formulations. According to this object of the present invention sucralose is present in an amount of between 0.001 to 2.00 % by weight, preferably it is 0.01 to 1.00 % by weight.

In a further embodiment the orally disintegrating tablet formulation of donepezil hydrochloride further comprises one or more pharmaceutically acceptable excipients selected from the group comprising lubricants, glidants and disintegrants.

Suitable lubricants may comprise but not limited to sodium stearyl fumarate, magnesium stearate, polyethylene glycol, stearic acid, metal stearates, boric acid, sodium chloride benzoate and acetate and the like and mixtures thereof, preferably the lubricant is sodium stearyl fumarate. In one aspect, sodium stearyl fumarate is present in an amount of 0.1 to 5.00 %, preferably it is 1.0 to 8.0 % by weight of the total tablet formulation.

Suitable glidants may comprise but not limited to colloidal silicon dioxide, calcium silicate, magnesium silicate and talc and the like and mixtures thereof, preferably the glidant is colloidal silicon dioxide. In one aspect, colloidal silicon dioxide is present in an amount of 0.01 to 5.00 %, preferably it is 0.1 to 5.0 % by weight of the total tablet formulation.

Suitable disintegrants other than L-HPC may comprise but not limited to microcrystalline cellulose, croscarmellose sodium, starch and pregelatinized starch and the like and mixtures thereof, preferably the second disintegrant is sodium starch glycolate. In one aspect, sodium starch glycolate is present in an amount of 0.1 to 10.0 % preferably it is 1.00 to 8.00 % by weight of the total tablet formulation.

As it is mentioned above, developing orally disintegrating compositions is difficult because of several different reasons. A satisfying orally disintegrating dosage form needs to fulfill the requirements of disintegration, taste and stability. To fulfill all these requirements the formulation for a specific drug needs to be adapted in particular by a careful selection of the excipients used.

In order to minimize the disintegration time and maximise the mechanical resistance of the tablets of this invention, this orally disintegrating tablet formulation has been designed, consisting the followings:
a. 1.00 to 10.0 % by weight of donepezil hydrochloride,
b. 1.00 to 15.00 % by weight of magnesium aluminum silicate,
c. 0.01 to 5.00 % by weight low viscosity HPC (HPC-SSL),
d. 1.00 to 20.00 % by weight high viscosity HPC (L-HPC),
e. 10.00 to 80.00 % by weight mannitol having the average particle size of 160 µm, (Pearlitol 160*C®*),
f. 5.00 to 50.00 % by weight mannitol having the average particle size of 360 µm, (*Pearlitol 400DC®*),
g. 0.001 to 2.00 % by weight sucralose,
h. 0.1 to 5.00 % by weight sodium stearyl fumarate,
i. 0.1 to 10.0 % by weight sodium starch glycolate,
j. 0.01 to 5.00 % by weight collodial silicon dioxide.

The process of the present invention for preparing the orally disintegrating tablet formulation of donepezil hydrochloride comprises the following steps;
a. mixing low viscosity HPC with a viscosity between 2.0 and 2.9 mPa.s and distilled water until having homogenous mixture,
b. mixing donepezil hydrochloride, mannitol having a volume average particle size of 160 µm, magnesium aluminum silicate and sucralose,
c. adding the granulation solution in (a) to the powder mixture in (b) and granulation process is conducted,
d. the wet granules are sieved and dried and and dried granules are sieved again,
e. adding mannitol having a volume average particle size of 360 µm, high viscosity HPC with a viscosity between 6.0 - 10.0 mPa.s, sodium starch glycolate, collodial silicon dioxide to the sieved granules and mixing,
f. adding the sieved sodium stearyl fumarate to the mixture in (e) and mixing until having homogenous mixture,
g. compressing the final mixture to form tablets.

In a further aspect, the present invention shows that it is possible to have a significant influence on the disintegration rate of the tablet by modifying the dimensions and shape of the tablet. In general, as the tablet becomes thinner and have higher porosity, the orally disintegrating composition will be weakened faster when it contacts with saliva, because the disintegration process is produced after wetting all the surface of the tablet via capillary action. Also, any shape which maximizes the contact surface with the saliva may produce a significant reduction in disintegration time. Therefore, the preferred shape of the orally disintegrating tablet is a round shape.

This invention is further defined by reference to the following examples. Although the examples are not intended to limit the scope of the present invention, it should be considered in the light of the description detailed above.

### Example 1 - Orally disintegrating donepezil hydrochloride tablets

| **Ingredients** | **Amount (mg)** |
|---|---|
| Donepezil hydrochloride | 10.00 |
| *Pearlitol 160C®* | 134.00 |
| Magnesium aluminum silicate | 12.60 |
| Sucralose | 1.5 |
| Hydroxypropyl cellulose (SSL) | 5.00 |
| Hydroxypropyl cellulose (LH-11) | 14.00 |
| *Pearlitol 400DC®* | 80.50 |
| Sodium starch glycolate | 14.00 |
| Collodial silicon dioxide | 1.40 |
| Sodium stearyl fumarate | 7.00 |
| Distilled water | k.m. |
| Total tablet weight | 280.0 |

### Example 2 - Orally disintegrating donepezil hydrochloride tablets

| Ingredients | Amount (mg) |
|---|---|
| Donepezil hydrochloride | 5.00 |
| *Pearlitol 160C®* | 139.00 |
| Magnesium aluminum silicate | 12.60 |
| Sucralose | 1.50 |
| Hydroxypropyl cellulose (SSL) | 5.00 |
| Hydroxypropyl cellulose (LH-11) | 14.00 |
| *Pearlitol 400DC®* | 80.50 |
| Sodium starch glycolate | 14.00 |
| Collodial silicon dioxide | 1.40 |
| Sodium stearyl fumarate | 7.00 |
| Distilled water | k.m. |
| Total tablet weight | 280.0 |

## Claims

1. The orally disintegrating tablet formulation of donepezil hydrochloride that is present in an amount of 1.00 to 10.0 % by weight comprising magnesium aluminum silicate and one or more pharmaceutically acceptable excipients, wherein magnesium aluminum silicate is present in an amount of 1.00 to 15.0% by weight of the total formulation and said formulation is crospovidon free.

2. The orally disintegrating tablet formulation of donepezil hydrochloride according to claim 1, further comprising hydroxypropyl cellulose with low and high viscosity; wherein the viscosity of low viscosity hydroxypropyl cellulose is between 2.0 and 2.9 mPa.s and the viscosity of high viscosity hydroxypropyl cellulose is between 6.0-10.0 mPa.s.

3. The orally disintegrating tablet formulation of donepezil hydrochloride according to claim 2, wherein the ratio of low viscosity hydroxypropyl cellulose with a viscosity between 2.0 and 2.9 mPa.s, to high viscosity hydroxypropyl cellulose with a viscosity between 6.0-10.0 mPa.s, is between 1:10 and 10:1 by weight.

4. The orally disintegrating tablet formulation of donepezil hydrochloride according to claims 1 to 3 comprising;
a. magnesium aluminum silicate in an amount of between 1.00 to 15.00 % by weight,
b. low viscosity hydroxypropyl cellulose with a viscosity between 2.0 and 2.9 mPa.s in an amount of between 0.01 to 5.00 % by weight,
c. high viscosity hydroxypropyl cellulose with a viscosity between 6.0-10.0 mPa.s in an amount of between 1.00 to 20.00 % by weight of total tablet formulation, and one or more pharmaceutically acceptable excipients.

5. The orally disintegrating tablet formulation of donepezil hydrochloride according to any of the preceding claims, further comprising two different mannitols, wherein one is having a volume average particle size of 160 µm and other mannitol is having a volume average particle size of 360 µm.

6. The orally disintegrating tablet formulation of donepezil hydrochloride according to claim 5, wherein the ratio of mannitol having a volume average particle size of 160 µm to mannitol having a volume average particle size of 360 µm is between 1:10 and 10:1 by weight.

7. The orally disintegrating tablet formulation of donepezil hydrochloride according to any of the preceeding claims, wherein the composition disintegrates in oral cavity in less than 60 seconds, preferably in less than 30 seconds.

8. The orally disintegrating tablet formulation of donepezil hydrochloride according to any of the preceding claims, wherein the hardness of the tablet is between 5 N to 50 N, preferably it is between 20 N to 30 N.

9. The orally disintegrating tablet formulation of donepezil hydrochloride according to any of the preceding claims consisting;
a. 1.0 to 10.0 % by weight of donepezil hydrochloride,
b. 1.00 to 15.00 % by weight of magnesium aluminum silicate,
c. 0.01 to 5.00 % by weight low viscosity hydroxypropyl cellulose with a viscosity between 2.0 and 2.9 mPa.s,
d. 1.00 to 20.00 % by weight high viscosity hydroxypropyl cellulose with a viscosity between 6.0-10.0 mPa.s,
e. 10.00 to 80.00 % by weight mannitol having a volume average particle size of 160 µm,
f. 5.00 to 50.00 % by weight mannitol having a volume average particle size of 360 µm,
g. 0.001 to 2.00 % by weight sucralose,
h. 0.1 to 5.00 % by weight sodium stearyl fumarate,
i. 0.1 to 10.0 % by weight sodium starch glycolate,
j. 0.01 to 5.00 % by weight collodial silicon dioxide.

10. A process for preparing orally disintegrating tablet formulation of donepezil hydrochloride according to any of the preceding claims comprising;
a. mixing low viscosity hydroxypropyl cellulose with a viscosity between 2.0 and 2.9 mPa.s and distilled water until having homogenous mixture,
b. mixing donepezil hydrochloride, mannitol having a volume average particle size of 160 µm, magnesium aluminum silicate and sucralose,
c. adding the granulation solution in (a) to the powder mixture in (b) and granulation process is conducted,
d. the wet granules are sieved and dried and dried granules are sieved again,
e. adding mannitol having a volume average particle size of 360 µm, high viscosity hydroxypropyl cellulose with a viscosity between 6.0-10.0 mPa.s, sodium starch glycolate, collodial silicon dioxide to the sieved granules and mixing,
f. adding the sieved sodium stearyl fumarate to the mixture in (e) and mixing until having homogenous mixture,
g. compressing the final mixture to form tablets.

## Patentansprüche

1. Im Mund zerfallende Tablettenformulierung von Donepezilhydrochlorid, das in einer Menge von 1,00 bis 10,0 Gew.-% vorliegt, umfassend Magnesiumaluminiumsilicat und einen oder mehrere pharmazeutisch verträgliche Exzipienten, wobei Magnesiumaluminiumsilicat in einer Menge von 1,00 bis 15,0 Gew.-% der gesamten Formulierung vorliegt und die Formulierung crospovidonfrei ist.

2. Im Mund zerfallende Tablettenformulierung von Donepezilhydrochlorid nach Anspruch 1, weiterhin umfassend Hydroxypropylcellulose mit niedriger und hoher Viskosität; wobei die Viskosität der niedrigviskosen Hydroxypropylcellulose zwischen 2,0 und 2,9 mPa·s beträgt und die Viskosität der hochviskosen Hydroxypropylcellulose zwischen 6,0-10,0 mPa·s beträgt.

3. Im Mund zerfallende Tablettenformulierung von Donepezilhydrochlorid nach Anspruch 2, wobei das Verhältnis von niedrigviskoser Hydroxypropylcellulose mit einer Viskosität zwischen 2,0 und 2,9 mPa·s zu hochviskoser Hydroxypropylcellulose mit einer Viskosität zwischen 6,0-10,0 mPa·s zwischen 1:10 und 10:1 nach Gewicht beträgt.

4. Im Mund zerfallende Tablettenformulierung von Donepezilhydrochlorid nach den Ansprüchen 1 bis 3, umfassend:
a. Magnesiumaluminiumsilicat in einer Menge von zwischen 1,00 bis 15,00 Gew.-%,
b. niedrigviskose Hydroxypropylcellulose mit einer Viskosität zwischen 2,0 und 2,9 mPa·s in einer Menge von zwischen 0,01 bis 5,00 Gew.-%,
c. hochviskose Hydroxypropylcellulose mit einer Viskosität zwischen 6,0-10,0 mPa·s in einer Menge von zwischen 1,00 bis 20,00 Gew.-% der gesamten Tablettenformulierung, und einen oder mehrere pharmazeutisch verträgliche Exzipienten.

5. Im Mund zerfallende Tablettenformulierung von Donepezilhydrochlorid nach einem der vorangehenden Ansprüche, weiterhin umfassend zwei unterschiedliche Mannitole, wobei das eine Mannitol eine volumengemittelte Teilchengröße von 160 µm aufweist und das andere Mannitol eine volumengemittelte Teilchengröße von 360 µm aufweist.

6. Im Mund zerfallende Tablettenformulierung von Donepezilhydrochlorid nach Anspruch 5, wobei das Verhältnis von Mannitol mit einer volumengemittelten Teilchengröße von 160 µm zu Mannitol mit einer volumengemittelten Teilchengröße von 360 µm zwischen 1:10 und 10:1 nach Gewicht beträgt.

7. Im Mund zerfallende Tablettenformulierung von Donepezilhydrochlorid nach einem der vorangehenden Ansprüche, wobei die Zusammensetzung in der Mundhöhle in weniger als 60 Sekunden, vorzugsweise in weniger als 30 Sekunden zerfällt.

8. Im Mund zerfallende Tablettenformulierung von Donepezilhydrochlorid nach einem der vorangehenden Ansprüche, wobei die Härte der Tablette zwischen 5 N bis 50 N beträgt, sie vorzugsweise zwischen 20 N bis 30 N beträgt.

9. Im Mund zerfallende Tablettenformulierung von Donepezilhydrochlorid nach einem der vorangehenden Ansprüche, bestehend aus
a. 1,0 bis 10,0 Gew.-% Donepezilhydrochlorid,
b. 1,00 bis 15,00 Gew.-% Magnesiumaluminiumsilicat,
c. 0,01 bis 5,00 Gew.-% niedrigviskoser Hydroxypropylcellulose mit einer Viskosität zwischen 2,0 und 2,9 mPa·s,
d. 1,00 bis 20,00 Gew.-% hochviskoser Hydroxypropylcellulose mit einer Viskosität zwischen 6,0-10,0 mPa·s,
e. 10,00 bis 80,00 Gew.-% Mannitol mit einer volumengemittelten Teilchengröße von 160 µm,
f. 5,00 bis 50,00 Gew.-% Mannitol mit einer volumengemittelten Teilchengröße von 360 µm,
g. 0,001 bis 2,00 Gew.-% Sucralose,
h. 0,1 bis 5,00 Gew.-% Natriumstearylfumarat,
i. 0,1 bis 10,0 Gew.-% Natriumstärkeglykolat,
j. 0,01 bis 5,00 Gew.-% kolloidalem Siliciumdioxid.

10. Verfahren zur Herstellung einer im Mund zerfallenden Tablettenformulierung von Donepezilhydrochlorid nach einem der vorangehenden Ansprüche, umfassend:
a. Mischen von niedrigviskoser Hydroxypropylcellulose mit einer Viskosität zwischen 2,0 und 2,9 mPa·s und destilliertem Wasser, bis ein homogenes Gemisch erhalten wird,
b. Mischen von Donepezilhydrochlorid, Mannitol mit einer volumengemittelten Teilchengröße von 160 µm, Magnesiumaluminiumsilicat und Sucralose,
c. Zugeben der Granulierungslösung in (a) zu dem Pulvergemisch in (b), und der Granulierungsprozess wird durchgeführt,
d. das feuchte Granulat wird gesiebt und getrocknet und das getrocknete Granulat wird noch einmal gesiebt,
e. Zugeben von Mannitol mit einer volumengemittelten Teilchengröße von 360 µm, hochviskoser Hydroxypropylcellulose mit einer Viskosität zwischen 6,0-10,0 mPa·s, Natriumstärkeglykolat, kolloidalem Siliciumdioxid zu dem gesiebten Granulat und Mischen,
f. Zugeben des gesiebten Natriumstearylfumarats zu dem Gemisch in (e) und Mischen, bis ein homogenes Gemisch erhalten wird,
g. Zusammenpressen des endgültigen Gemisches, um Tabletten zu formen.

## Revendications

1. Formulation de comprimé à désintégration orale de chlorhydrate de donépézil qui est présent dans une quantité de 1,00 à 10,0 % en poids, comprenant du silicate de magnésium et d'aluminium et un ou plusieurs excipients pharmaceutiquement acceptables, dans laquelle le silicate de magnésium et d'aluminium est présent dans une quantité de 1,00 à 15,0 % en poids de la formulation totale et ladite formulation est exempte de crospovidone.

2. Formulation de comprimé à désintégration orale de chlorhydrate de donépézil selon la revendication 1, comprenant en outre de l'hydroxypropyl cellulose à faible viscosité et de l'hydroxypropyl cellulose à viscosité élevée, dans laquelle la viscosité de l'hydroxypropyl cellulose à faible viscosité est entre 2,0 et 2,9 mPa.s et la viscosité de l'hydroxypropyl cellulose à viscosité élevée est entre 6,0 et 10,0 mPa.s.

3. Formulation de comprimé à désintégration orale de chlorhydrate de donépézil selon la revendication 2, dans laquelle le rapport de l'hydroxypropyl cellulose à faible viscosité ayant une viscosité entre 2,0 et 2,9 mPa.s, à l'hydroxypropyl cellulose à viscosité élevée ayant une viscosité entre 6,0 et 10,0 mPa.s, est entre 1:10 et 10:1 en poids.

4. Formulation de comprimé à désintégration orale de chlorhydrate de donépézil selon l'une des revendications 1 à 3 comprenant :
a - du silicate de magnésium et d'aluminium dans une quantité d'entre 1,00 et 15,00 % en poids ;
b - de l'hydroxypropyl cellulose à faible viscosité ayant une viscosité entre 2,0 et 2,9 mPa.s dans une quantité d'entre 0,01 et 5,00 % en poids ;
c - de l'hydroxypropyl cellulose à viscosité élevée ayant une viscosité entre 6,0 et 10,0 mPa.s dans une quantité d'entre 1,00 et 20,00 % en poids de la formulation de comprimé totale, et
un ou plusieurs excipients pharmaceutiquement acceptables.

5. Formulation de comprimé à désintégration orale de chlorhydrate de donépézil selon l'une quelconque des revendications précédentes, comprenant en outre deux différents mannitols, dans laquelle l'un est un mannitol ayant une dimension de particule moyenne en volume de 160 µm et l'autre mannitol est un mannitol ayant une dimension de particule moyenne en volume de 360 µm.

6. Formulation de comprimé à désintégration orale de chlorhydrate de donépézil selon la revendication 5, dans laquelle le rapport du mannitol ayant une dimension de particule moyenne en volume de 160 µm au mannitol ayant une dimension de particule moyenne en volume de 360 µm est entre 1:10 et 10:1 en poids.

7. Formulation de comprimé à désintégration orale de chlorhydrate de donépézil selon l'une quelconque des revendications précédentes, dans laquelle la composition se désintègre dans la cavité orale en moins de 60 secondes, de préférence en moins de 30 secondes.

8. Formulation de comprimé à désintégration orale de chlorhydrate de donépézil selon l'une quelconque des revendications précédentes, dans laquelle la dureté du comprimé est entre 5 N et 50 N, étant de préférence entre 20 N et 30 N.

9. Formulation de comprimé à désintégration orale de chlorhydrate de donépézil selon l'une quelconque des revendications précédentes, consistant en :
a - 1,0 à 10,0 % en poids de chlorhydrate de donépézil ;
b - 1,00 à 15,00 % en poids de silicate de magnésium et d'aluminium,
c - 0,01 à 5,00 % en poids d'hydroxypropyl cellulose à faible viscosité ayant une viscosité entre 2,0 et 2,9 mPa.s ;
d - 1,00 à 20,00 % en poids d'hydroxypropyl cellulose à viscosité élevée ayant une viscosité entre 6,0 et 10,0 mPa.s ;
e - 10,00 à 80,00 % en poids de mannitol ayant une dimension de particule moyenne en volume de 160 µm ;
f - 5,00 à 50,00 % en poids de mannitol ayant une dimension de particule moyenne en volume de 360 µm ;
g - 0,001 à 2,00 % en poids de sucralose ;
h - 0,1 à 5,00 % en poids de fumarate de sodium et de stéaryle ;
i - 0,1 à 10,0 % en poids de glycolate d'amidon sodique ;
j - 0,01 à 5,00 % en poids de dioxyde de silicium colloïdal.

10. Procédé de préparation d'une formulation de comprimé à désintégration orale de chlorhydrate de donépézil selon l'une quelconque des revendications précédentes, comprenant :
a - mélanger de l'hydroxypropyl cellulose à faible viscosité ayant une viscosité entre 2,0 et 2,9 mPa.s et de l'eau distillée jusqu'à avoir un mélange homogène ;
b - mélanger du chlorhydrate de donépézil, du mannitol ayant une dimension de particule moyenne en volume de 160 µm, du silicate de magnésium et d'aluminium et du sucralose ;
c - ajouter la solution de granulation obtenue en (a) au mélange pulvérulent obtenu en (b) et un procédé de granulation est conduit ;
d - les granulés humides sont tamisés et séchés et les granulés séchés sont tamisés à nouveau ;
e - ajouter du mannitol ayant une dimension de particule moyenne en volume de 360 µm, de l'hydroxypropyl cellulose à viscosité élevée ayant une viscosité entre 6,0 et 10,0 mPa.s, du glycolate d'amidon sodique, de dioxyde de silicium colloïdal aux granulés tamisés, et mélanger ;
f - ajouter le fumarate de sodium et de stéaryle tamisé au mélange obtenu en (e) et mélanger jusqu'à avoir un mélange homogène ;
g - comprimer le mélange final pour former des comprimés.
